# EUROPEAN PATENT SPECIFICATION

(11) **EP 4 475 962 B1**
(45) Date of publication and mention of the grant of the patent: **06.08.2025**
(21) Application number: 23703445.9
(22) Date of filing: 07.02.2023
(51) Int. Cl.: A61Q 19/10, A61K 8/02, A61K 8/36, A61K 8/368, A61K 8/37, A61K 8/44, A61K 8/46, A61K 8/73

(54) **DRY COMPOSITIONS AND METHODS FOR FORMING STABLE LIQUID CLEANSING FORMULATIONS**
TROCKENE ZUSAMMENSETZUNGEN UND VERFAHREN ZUR HERSTELLUNG VON STABILEN FLÜSSIGEN REINIGUNGSFORMULIERUNGEN
COMPOSITIONS SÈCHES ET PROCÉDÉS POUR LA FORMATION DE FORMULES DE NETTOYAGE LIQUIDES STABLES

(30) Priority: 08.02.2022 EP 22155623
(43) Date of publication of application: 18.12.2024
(73) Proprietor: Unilever IP Holdings B.V., 3013 AL Rotterdam (NL); Unilever Global IP Limited, Wirral, Merseyside CH62 4ZD (GB)
(72) Inventor: AKRE, Himanshu, 6708 WH Wageningen (NL); BAPAT, Mohini Anand, 6708 WH Wageningen (NL); HAQUE, Aaliya, 6708 WH Wageningen (NL); KUMAR, Nitish, 6708 WH Wageningen (NL); SAYYED, Jabir Gulab, 6708 WH Wageningen (NL); TOMAR, Nikita, 6708 WH Wageningen (NL)
(74) Representative: Askew, Sarah Elizabeth
(86) International application number: PCT/EP2023/052946
(87) International publication number: WO 2023/152112

(56) References cited:
- EP-B1- 2 500 012
- CN-A- 106 619 183
- FR-A1- 2 984 155
- US-A1- 2018 193 231
- US-A1- 2019 029 936
- US-B2- 9 271 919

## Description

### Field of the invention

The present invention relates to cleansing compositions. The invention particularly relates to dry compositions and methods for forming stable liquid personal cleansing formulations therefrom. More particularly the invention relates to powder-based compositions which are capable of being reconstituted with a suitable liquid carrier.

### Background of the invention

The invention has been developed primarily for use in personal wash application and will be described hereinafter with reference to this application. However, it will be appreciated that the invention is not limited to this particular field of use.

Any discussion of the prior art throughout the specification should in no way be considered as an admission that such prior art is widely known or forms part of the common general knowledge in the field.

Personal cleansing compositions are available in wide ranging product formats e.g. from soap bars, liquid soap, body-wash compositions and self-foaming compositions to shampoos. Sometimes such cleansing compositions are also included in cleansing wipes.

Liquid cleansing formulations have been known since long. There is a growing consumer demand for refill packs of such liquid formulations, however the refill packs do not fully solve the problems or expenses associated with the bottled liquid formulations such that cost of packaging and handling and transport difficulties.

As an alternative dry powdered compositions which can be reconstituted to obtain liquid formulations by consumer are a feasible option which can reduce the costs as well as avoid handling and transporting issues. Nonetheless, such composition also have problems such as shelf life, appearance of the reconstituted liquid formulation, time of reconstitution, uneven reconstitution, separation of phases, settling of gel.

The documents US-A-2019/029936, US-A-2018/193231 or CN-A-106619183 disclose such dry powdered compositions suitable for forming liquid cleansing compositions.

Therefore, there is a need for a dry composition which solves these issues.

### Summary of the invention

According to a first aspect, present invention discloses a dry composition comprising: 30 to 90 wt% of surfactant; 1 to 6 wt% of chelating agent; 0.5 to 20 wt% of preservative; and cellulosic polymer, wherein at most 50 wt% of the total weight of cellulosic polymer has degree of substitution less than 0.7.

A liquid formulation is obtained by reconstituting the dry composition according to the first aspect with a suitable liquid carrier, the liquid composition comprising: 0.5 to 9 wt% of surfactant; 0.01 to 0.6wt% of chelating agent; 0.01 to 2 wt% preservative; cellulosic polymer, wherein at most 50 wt% of the total weight of cellulosic polymer has degree of substitution less than 0.7; and 85 to 97 wt% liquid carrier.

Disclosed is also the use of dry composition according to the first aspect to obtain a liquid formulation.

Second aspect of the present invention provides a method of making a liquid formulation from the dry composition according to the first aspect, the method comprising steps of, taking the dry composition and a container and diluting the dry composition with a liquid carrier with a dilution factor in the range of 1:10 to 1:50 to obtain the liquid formulation.

These and other aspects, features and advantages will become apparent to those of ordinary skill in the art from a reading of the following detailed description and the appended claims. For the avoidance of doubt, any feature of one aspect of the present invention may be utilized in any other aspect of the invention.

The term "comprising" is meant not to be limiting to any subsequently stated elements but rather to encompass non-specified elements of major or minor functional importance. In other words the listed steps, elements or options need not be exhaustive. Whenever the words "including" or "having" are used, these terms are meant to be equivalent to "comprising" as defined above. In other words, the listed steps or options need not be exhaustive It is noted that the examples given in the description below are intended to clarify the invention and are not intended to limit the invention to those examples per se. Similarly, all percentages are weight/weight percentages unless otherwise indicated.

Numerical ranges expressed in the format "from x to y" are understood to include x and y. When for a specific feature multiple preferred ranges are described in the format "from x to y", it is understood that all ranges combining the different endpoints are also contemplated.

It should be noted that in specifying any range of concentration or amount, any particular upper concentration can be associated with any particular lower concentration or amount.

For a more complete understanding of the above and other features and advantages of the invention, reference should be made to the following detailed description of preferred embodiments.

### Detailed description of the invention

The present invention relates to dry compositions and methods for forming stable liquid personal cleansing formulations. More particularly the invention relates to powder-based compositions which are capable of being reconstituted with a suitable liquid carrier.

The present inventors have been able to formulate a dry compositions with selected components such that the resultant compositions are readily solubilized and stable in liquid carriers. Further the dry compositions upon reconstitution in suitable liquid carriers result in liquid formulations having desired rheology, viscosity, transparency and does not show any gel settling, separation. Further, the liquid formulations obtained from reconstitution of the dry compositions allow for easier delivery of the dry compositions and increased shelf stability.

### Dry Composition

The dry compositions of the present invention are preferably in powder form, the formulation may be granular. The dry compositions of the present invention are made with an objective of being reconstituted with a suitable liquid carrier to form stable liquid formulations which are preferably used a cleansing compositions and more preferably personal wash cleansing compositions.

Hereinafter, the terms cellulosic polymer and cellulosic gum may be used interchangeably. Hereinafter, the cellulosic polymer having degree of substitution higher than 0.75 will be termed as higher degree of substitution (HDS) and the cellulosic polymer having degree of substitution less than 0.7 will be termed as lower degree of substitution (LDS).

The dry composition of the present invention suitable for forming stable liquid cleansing compositions comprises:
i) 30 to 90 wt% of surfactant;
ii) 1 to 6 wt% of chelating agent;
iii) 0.5 to 20 wt% preservative; and
iv) cellulosic polymer, wherein at most 50 wt% of the total weight of cellulosic polymer has degree of substitution less than 0.7.

It is preferred that in the dry composition the surfactant is the range of 40 to 80 wt% of the dry composition. It is preferred that the surfactant is an anionic surfactant.

It is preferred that in the dry composition the surfactant comprises co-surfactants in the range of 0 to 20 wt% of the dry composition. It is preferred that the co-surfactant is an anionic surfactant.

It is preferred that in the dry composition the chelating agent is in the range of 1.5 to 5.5 wt%, preferably the chelating agent is Ethylenediaminetetraacetic acid (EDTA) or diethylenetriaminepentaacetic acid (DTPA), mixtures or combinations thereof It is preferred that in the dry composition the preservative is in the range of 1.5 to 18wt% of the dry composition. It is preferred that the preservative is benzoic acid or derivative, paraben derivative, or a sorbate derivative or mixtures or combinations thereof. More preferably the preservative is selected from sodium benzoate, benzoic acid, potassium sorbate, methylparaben, ethylparaben, propylparaben, butylparaben and heptyl paraben, combinations or mixtures thereof.

It is preferred that in the dry composition the cellulosic polymer is in the range of 5 to 30 wt% of the dry composition, more preferably the cellulosic polymer is a carboxy methyl derived cellulose.

It is further preferred that the cellulosic polymer having degree of substitution less than 0.7 and the cellulosic polymer having degree of substitution higher than 0.75, is in the range of 1:1.5 to 1:4.

### Surfactant

The present invention comprises a surfactant. The surfactant may be a soap or a non-soap surfactant. The compositions of the present invention may contain anionic surfactants, nonionic surfactants, cationic surfactants or amphoteric surfactants.

The surfactant in the dry composition is present in the range of 30 to 90 wt% by weight of the dry composition, preferably in the range of 40 to 80 wt% by weight of the dry composition, further preferably in the range of 45 to 75 wt% by weight of the dry composition and most preferably in the range of 50 to 70 wt% by weight of the dry composition.

The surfactant in the dry composition is present at least 30wt%, preferably at least 40wt%, further preferably at least 45wt% and most preferably at least 50wt% by weight of the dry composition.

The surfactant in the dry composition is present at most 90wt%, preferably at most 80wt%, further preferably at most 75wt% and most preferably at most 70wt% by weight of the dry composition.

Preferably the anionic surfactants for the purposes of the present invention are, for example, acylamino acids and salts thereof, such as acylglutamates, in particular sodium acyl glutamate sarcosinates, for example myristoyl sarcosine, TEA-iauroyl sarcosinate, sodium lauryl sarcosinate and sodium cocoayl sarcosinate. Sulfonic acids and their salts, such as Acylisethionates, z.B. sodium / ammonium cocoyl isethionate, sulfosuccinates, for example dioctyl sodium sulfosuccinate, disodium laureth sulfo- succinate, disodium lauryl sulfosuccinate and disodium undecylenamido MEA- sulfosuccinate; and sulfur acids, such as alkyl ether sulfate, for example sodium, ammonium, magnesium, MIPA, TIPA laureth sulfate, sodium myreth sulfate and sodium C12-13 pareth sulphate, alkyl sulfates, for example sodium, ammonium and TEA lauryl sulfate. Furthermore, taurates, for example sodium lauroyl taurate and sodium methylcocoyl taurate, ether carboxylic acids, for example sodium laureth-13 carboxylate and sodium PEG-6 cocamide carboxylate, phosphoric acid esters and salts, such as DEA-oleth-10 phosphate and dilaureth-4 phosphate, alkylsulfonates, for example sodium cocosmonoglyceride sulfate, sodium C12-ₗ Olefin sulfonate, sodium lauryl sulfoacetate and magnesium PEG-3 cocamide sulphate. Acylglutamates such as di-TEA-palmitoylaspartate and sodium caprylic/ capric glutamate, acyl peptides, for example palmitoyl, hydrolyzed milk protein, sodium cocoyl hydrolyzed soy protein and sodium/ potassium cocoyl hydrolyzed collagen as well as carboxylic acids and derivatives, such as lauric acid, aluminum stearate, magnesium alkanolate and tincient cylenate, ester carboxylic acids, for example calcium stearoyl lactylate, laureth-6 citrate and sodium PEG-4 lauramid carboxylate, alkylarylsulfonate.

The most preferred anionic surfactants for the compositions of the present invention are selected from the group of sulfonic acids, their salts; alkyl ether sulfates, ammonium lauryl sulfate, sodium laureth sulfate, sodium lauryl sarcosinate, sodium laurethl sarcosinate , Disodium Lauryl Sulfosuccinate, sodium myreth sulfate, sodium pareth sulfate, sodium lauryl sulfate, alpha olefin sulfonate, ammonium laureth sulfate, mixtures and combinations thereof.

The anionic surfactant in the dry composition is present in the range of 30 to 90 wt% by weight of the dry composition, preferably in the range of 40 to 80 wt% by weight of the dry composition, further preferably in the range of 45 to 75 wt% by weight of the dry composition and most preferably in the range of 50 to 70 wt% by weight of the dry composition.

It is preferred that the anionic surfactant in the dry composition is present at least 30wt%, more preferably at least 40wt%, further preferably at least 45wt% and most preferably at least 50wt% by weight of the dry composition.

The anionic surfactant in the dry composition is present at most 90wt%, mere preferably at most 80wt%, further preferably at most 75wt% and most preferably at most 70wt% by weight of the dry composition.

It is highly preferred that the surfactant of the present composition is at least 70wt% anionic surfactant, more preferably at least 80 wt% and most preferably at least 90 wt% of the total weight of total surfactant

Preferably the cationic surfactants for the purposes of the present invention are, for example, qúarternary surfactants, these include, but are not limited to: benzalkonium chloride, alkyl betaine, alkylamidopropyl betaine, alkyl-amidopropylhydroxysultaine alkylamines, alkylimidazoles and ethoxylated amines.

Preferably the amphoteric surfactants for the purposes of the present invention are, for example, acyl / dialkylethylene diamines, for example sodium acylamphoacetate, disodium acyl amphodipropionate, disodium alkylamphodiacetate, sodium acrylamphohydroxypropyl sulfonate, disodium acrylamphodiacetate and sodium acrylamphopropionate, N-alkylamino acids, for example aminopropyl alkylglutamide, alkylaminopropionic acid, sodium alkylimidodipropionate and lauroamphocarboxyglycinate.

Preferably the non-ionic surfactants for the purposes of the present invention are, for example, Alkanolamides, such as Cocamide MEA DEA/ MIPA, esters produced by the esterification of carboxylic acids with ethylene oxide, glycerol, sorbitan or other alcohols, ethers, for example ethoxylated alcohols, ethoxylated lanolin, ethoxylated poly-siloxanes, propoxylated POE ethers and alkyl polyglycosides such as lauryl glucoside, decyl glycoside and cocoglycoside.

Suitable surfactants that may be used in the formulations disclosed herein may comprise one or more of sodium cocoyl isethionate, disodium lauryl sulfosuccinate, lauryl glucoside, myristyl glucoside, sodium sulfate, sodium silicate, sodium coco sulfate, sodium lauryl sulfate. In one embodiment, the surfactant may comprise sodium cocoyl isethionate (e.g., Jordapon^{®} SCI powder). In another embodiment, the surfactant may comprise disodium lauryl sulfosuccinate (e.g., Plantapon^{®} SUS). In a further embodiment, the surfactant may comprise a mixture of lauryl glucoside, myristyl glucoside, sodium sulfate, sodium silicate, and sodium coco sulfate (e.g., Glucopol^{®} 50 G). In yet another embodiment, the surfactant may comprise sodium lauryl sulfate (e.g., Texapon^{®} Z-95 P). Suitable surfactants are not limited to the ones enumerated herein and may include other surfactants that are in powder form prior to their incorporation into the formulation and are water-soluble to maximize the cleansing potential of the formulation.

### Co-surfactants

The compositions of the present invention may comprise a co-surfactant. It is preferred that the present composition comprises co-surfactants in the range of 0 to 20wt%, i.e, up to 20 wt%, or at most 20 wt%, preferably 0.01 to 20 wt%, more preferably 2 to 18 wt% and most preferably 5 to 15 wt%.

Co-surfactants for the purposes of the present invention may be anionic, cationic, amphoteric, non-ionic or zwitterionic, mixtures or combinations thereof.

A zwitterionic surfactant can be a co-surfactant selected from the group consisting of: lauryl hydroxysultaine, cocamidopropyl hydroxysultaine, coco-betaine, coco-hydroxysultaine, coco-sultaine, lauryl betaine, lauryl sultaine, and mixtures thereof.

The non-ionic co-surfactant can be selected from the group consisting alkyl polyglucoside, alkyl glycoside, acyl glucamide and mixture thereof. Non-limiting examples of alkyl glucosides can include decyl glucoside, cocoyl glucoside, lauroyl glucoside and combination thereof.

When the compositions of the present invention comprise a co-surfactant, it is preferred to use an anionic co-surfactant. The anionic co-surfactant can be selected from the group consisting of isethionates, sarcosinates, sulfosuccinates, sulfonates, sulfoacetates, glucosides, acyl glycinates, acyl alaninates, glucose carboxylates, amphoacetates, taurates, and mixture thereof.

Suitable isethionate surfactants can include the reaction product of fatty acids esterified with isethionic acid and neutralized with sodium hydroxide. Suitable fatty acids for isethionate surfactants can be derived from coconut oil or palm kernel oil including amides of methyl tauride. Non-limiting examples of isethionates can be selected from the group consisting of sodium lauroyl methyl isethionate, sodium cocoyl isethionate, ammonium cocoyl isethionate, sodium hydrogenated cocoyl methyl isethionate, sodium lauroyl isethionate, sodium cocoyl methyl isethionate, sodium myristoyl isethionate, sodium oleoyl isethionate, sodium oleyl methyl isethionate, sodium palm kerneloyl isethionate, sodium stearoyl methyl isethionate, and mixtures thereof.

Non-limiting examples of sarcosinates can be selected from the group consisting of sodium lauroyl sarcosinate, sodium cocoyl sarcosinate, sodium myristoyl sarcosinate, TEA-cocoyl sarcosinate, ammonium cocoyl sarcosinate, ammonium lauroyl sarcosinate, dimer dilinoleyl bis-lauroylglutamate/lauroylsarcosinate, disodium lauroamphodiacetate lauroyl sarcosinate, isopropyl lauroyl sarcosinate, potassium cocoyl sarcosinate, potassium lauroyl sarcosinate, sodium cocoyl sarcosinate, sodium lauroyl sarcosinate, sodium myristoyl sarcosinate, sodium oleoyl sarcosinate, sodium palmitoyl sarcosinate, TEA-cocoyl sarcosinate, TEA-lauroyl sarcosinate, TEA-oleoyl sarcosinate, TEA-palm kernel sarcosinate, and combinations thereof.

Non-limiting examples of sulfosuccinate surfactants can include disodium N-octadecyl sulfosuccinate, disodium lauryl sulfosuccinate, diammonium lauryl sulfosuccinate, sodium lauryl sulfosuccinate, disodium laureth sulfosuccinate, tetrasodium N-(1,2-dicarboxyethyl)-N-octadecyl sulfosuccinnate, diamyl ester of sodium sulfosuccinic acid, dihexyl ester of sodium sulfosuccinic acid, dioctyl esters of sodium sulfosuccinic acid, and combinations thereof.

Non-limiting examples of sulfonates can include alpha olefin sulfonates, linear alkylbenzene sulfonates, sodium laurylglucosides hydroxypropylsulfonate and combination thereof.

Non-limiting examples of sulfoacetates can include sodium lauryl sulfoacetate, ammonium lauryl sulfoacetate and combination thereof.

Non-limiting example of acyl glycinates can include sodium cocoyl glycinate, sodium lauroyl glycinate and combination thereof.

Non-limiting example of acyl alaninates can include sodium cocoyl alaninate, sodium lauroyl alaninate, sodium N-dodecanoyl-1-alaninate and combination thereof.

Non-limiting example of glucose carboxylates can include sodium lauryl glucoside carboxylate, sodium cocoyl glucoside carboxylate and combinations thereof.

Non-limiting example of alkyl ether carboxylate can include sodium laureth-4 carboxylate, laureth-5 carboxylate, laureth-13 carboxylate, sodium C12-13 pareth-8 carboxylate, sodium C12-15 pareth-8 carboxylate and combination thereof.

Non-limiting example of alkylamphoacetates can include sodium cocoyl amphoacetate, sodium lauroyl amphoacetate and combination thereof.

Non-limiting example of acyl taurates can include sodium methyl cocoyl taurate, sodium methyl lauroyl taurate, sodium methyl oleoyl taurate and combination thereof.

### Chelating Agent

The present invention comprises a chelating agent. The chelating agent in the dry composition is present in the range of 1 to 6 wt% by weight of the dry composition, preferably in the range of 1.5 to 5.5 wt% by weight of the dry composition, further preferably in the range of 2 to 5 wt% by weight of the dry composition and most preferably in the range of 2.5 to 4.8 wt% by weight of the dry composition.

The chelating agent in the dry composition is present at least 1wt%, preferably at least 1.5 wt%, further preferably at least 2 wt% and most preferably at least 2.8 wt% by weight of the dry composition.

The chelating agent in the dry composition is present at most 6 wt%, preferably at most 5.5 wt%, further preferably at most 5 wt% and most preferably at most 4.8 wt% by weight of the dry composition.

The preferred chelating agents are as follows (names followed by their abbreviation in parenthesis):
Ethylene Diamine Tetra Acetic acid (EDTA), Diethylene Triamine Penta Acetic acid (DTPA), Ethane-1 -hydroxy-1 ,1-diphosphonate (EHDP), Ethylene Diamine-N,N'-Disuccinate (EDDS), Nitrilo Triacetic Acid (NTA), Sodium Imino Disuccinate (IDS), Ethylene Glycol-bis-(2-aminoethyl)-N,N,N', N'-Tetra Acetic acid (EGTA), Methyl Glycine Diacetic Acid (MGDA), N-(2-hydroxyethyl) Ethylene Diamine N,N',N'-Thacetic acid) (HEDTA), Ethylene Diamine Tetra Methylene Phosphonic acid (EDTMP), Diethylene Thamine-Penta-Methylene Phosphonic acid (DTPMP), Glutamic acid-N,N-Diacetic Acid (GLDA), Cyclohexane-1 ,2-Diamine-N,N,N',N'-Tetra-Acetic Acid (CDTA), 1 ,3-Propylenediamine Tetra-Acetic Acid (PDTA), Ethylene Diamine Triacetic Acid (EDTA), L-hydroxy Imino Disuccinic acid (L-IDS), Trisodium N-Carboxyethyl Imino Succinate (CEIS), Citric Acid, Sodium Thpolyphosphate (STP), Thethylene Tetramine Hexaacetic Acid (TTHA). Other preferred chelating agents are Trisodium Ethylene Diamine Disuccinate, Tetra-sodium-Imino disuccinate, Glutamic acid-N,N diacetic acid tetra sodium salt, 2-hydroxyethyl iminodiacetic acid, Sodium salt (disodium ethanol diglycinate), Tetrasodium 3-hydroxy-2,2 imino disuccinate, Trisodium methylglycine diacetic acid, L-Aspartate-N,N-diacetic acid tetrasodium salt. The more preferred chelating agents are salt of Ethylene Diamine Tetra Acetic acid (EDTA) and salt of Diethylene Thamine Penta Acetic acid (DTPA). Preferred salts of EDTA are disodium Ethylene Diamine Tetra Acetic acid and tetrasodium Ethylene Diamine Tetra Acetic acid. Preferred salt of DTPA is the pentasodium Diethylene Thamine Penta Acetic acid.

The salt form is preferred over acid form, as addition of the chelating agents in the acid form would bring about a corresponding decrease in pH, which is not desirable.

It is most preferred that the chelating agent suitable for the compositions of the present invention are selected from the group of Ethylene Diamine Tetra Acetic acid (EDTA), or Diethylene Triamine Penta Acetic acid (DTPA), their derivatives and combinations, mixtures thereof. The EDTA derivative is selected from tetrasodium EDTA, trisodium EDTA, disodium EDTA, and combinations thereof. The DTPA derivative is selected from tetrasodium DTPA, trisodium DTPA, disodium DTPA, and combinations thereof.

### Preservative

The present invention comprises a preservative. The preservative in the dry composition is present in the range of 0.5 to 20 wt% by weight of the dry composition, preferably in the range of 1.5 to 19 wt% by weight of the dry composition, further preferably in the range of 2 to 18 wt% by weight of the dry composition and most preferably in the range of 3 to 17 wt% by weight of the dry composition.

The preservative in the dry composition is present at least 0.5wt%, preferably at least 1.5 wt%, further preferably at least 2 wt% and most preferably at least 3 wt% by weight of the dry composition.

The preservative in the dry composition is present at most 20 wt%, preferably at most 19 wt%, further preferably at most 18 wt% and most preferably at most 17 wt% by weight of the dry composition.

Very useful examples of the preservatives suitable for the compositions of the present inventions, but are not limited to, chlorite components, sorbic acid components and mixtures thereof.

Specific examples of chlorite components useful as preservatives in accordance with the present invention include stabilized chlorine dioxide (SCD), metal chlorites, such as alkali metal and alkaline earth metal chlorites, and mixtures therefor. Technical grade (or USP grade) sodium chlorite is a very useful preservative component. The exact chemical composition of many chlorite components, for example, SCD, is not completely understood. Specific examples of sorbic acid components useful as preservatives in accordance with the present invention include sorbic acid itself, as well as pharmaceutically and/or ophthalmically acceptable sorbic acid derivatives and mixture thereof. Useful sorbic acid components include, but are not limited to, metal sorbates, such as alkali metal and alkaline earth metal sorbates, and mixtures thereof.

Other preferred preservatives include but not limited to commonly used in cosmetics, such as dibromdicyanobutane (2-bromo-2-bromomethylglutarodinitrile), phenoxyethanol, 3-lod-2-propinylbutylcarbamate, 2-bromo-2-nitro-propane-1, 3-diol, imidazolidinyl hamstoff, 5-chloro-2-methyl-4-isothiazolin-3-one, 2-chloroacetamide, benzalkonium chloride, benzyl alcohol.' It is particularly preferred according to the invention if sodium benzoate, sodium salicylate, methyldibromoglutaronitrile and / or phenoxyethanol are used as preservatives.

### Cellulosic polymer

The compositions of the present invention comprise a cellulosic polymer. The polymer is preferably used as a thickening agent. Any other thickening agent may also be used which serves the same purpose.

The cellulose based thickening agent according to the present invention is selected from hydroxyl ethyl cellulose, hydroxyl methyl cellulose, hydroxyl propyl cellulose, hydroxyl propyl methyl cellulose, carboxy methyl cellulose and methyl cellulose.

It is preferred that the cellulosic polymer in the dry composition is present in the range of 5 to 30 wt% by weight of the dry composition, more preferably in the range of 7 to 25 wt% by weight of the dry composition, further preferably in the range of 10 to 23 wt% by weight of the dry composition and most preferably in the range of 10 to 20 wt% by weight of the dry composition.

It is preferred that the cellulosic polymer in the dry composition is present at least 5wt%, more preferably at least 7 wt%, further preferably at least 10 wt% and most preferably at least 11 wt% by weight of the dry composition.

It is preferred that the cellulosic polymer in the dry composition is present at most 30 wt%, more preferably at most 25 wt%, further preferably at most 23 wt% and most preferably at most 20 wt% by weight of the dry composition.

The present invention comprises a nonionic or anionic water-soluble polymer. Suitable nonionic polymers include such water soluble polymers as cellulose ethers (e.g., hydroxybutyl methylcellulose, hydroxypropylcellulose, hydroxypropyl methylcellulose, ethylhydroxy ethylcellulose and hydroxyethylcellulose), propylene glycol alginates, polyacrylamide, poly(ethylene oxide), polyvinyl alcohol, polyvinylpyrrolidone, hydroxypropyl guar gum, locust bean gum, amylose, hydroxyethyl amylose, starch and starch derivatives and mixtures thereof. Preferred nonionic polymers include hydroxyethyl cellulose, polyethylene oxide, polyvinyl pyrrolidone, polyvinyl alcohol, polyacrylamide, hydroxypropyl cellulose, ethylhydroxy ethyl cellulose, dextran, polypropyleneoxide and hydroxypropyl guar or mixtures thereof. Suitable anionic water-soluble polymers include carboxymethyl cellulose, carrageenan, xanthum gum polystyrene sulfonate, gum agar, gum ghatti, gum karaya, pectins, alginate salts, as well as poly(acrylic acid) and acrylic or methacrylic acid derivatives such as the alkali metal and ammonium salts of acrylic acid, methacrylic acid. Mixtures of the above anionic water-soluble polymers may also be used.

These polymeric compositions may be homopolymers or they may be copolymers or terpolymers with other copolymerizing monomers known in the art. Examples of copolymerizing monomers known in the art include but are not limited to ethylene, propylene, isobutylene, styrene, polystyrene, alphamethylstyrene, vinyl acetate, vinyl formate, alkyl ethers, acrylonitrile, methacrylonitrile, vinyl chloride, vinylidene chloride, the alkyl acrylates, the alkylmethacrylates, the alkyl fumarates, the alkyl maleates, and other olefinic monomers copolymerizable therewith as long as the resulting polymers are water soluble and phase separate in the compositions of this invention. Copolymers of anionic and nonionic monomers such as acrylic acid and methacrylic acid with acrylamide. methacrylamide, the N-alkyl substituted amides, the N-aminoalkylamides, the corresponding N-alkylaminoalkyl substituted amides, the aminoalkyl acrylates, the aminoalkyl methacrylamides, and the N-alkyl substituted aminoalkyl esters of either acrylic or methacrylic acids.

Preferred anionic polymers include polyacrylic acid; sodium carboxy methyl cellulose; polyacrylates; polymethyl acrylate; polysulphates such as polyvinyl sulfate, polystyrene sulfonate, polyphosphates, sodium dextran sulfate, alginate salts and pectate When combined with the aqueous surfactant system and phase separation initiator, described below, the water-soluble nonionic or anionic polymer separates to form aqueous droplets suspended in a continuous aqueous phase. The number average particle size of the polymer droplets can be from 0.1 microns to about 10,000 microns, preferably from about 1.0 micron to about 5000 microns, most preferably from about 5 microns to about 1000 microns.

Most preferred for use in the present invention are selected from the list of carboxymethyl cellulose (CMC), cellulose gum, cellulose derivative, its salt form, combinations and mixtures thereof.

It is highly preferred to use cellulose gum as the polymer for the compositions of the present invention. It is highly preferred to use anionic water soluble polymers such as carboxymethyl cellulose, carrageenan, xanthum gum polystyrene sulfonate, gum agar, gum ghatti, gum karaya, pectins, alginate salts, as well as poly(acrylic acid) and acrylic or methacrylic acid derivatives such as the alkali metal and ammonium salts of acrylic acid, methacrylic acid, and mixtures thereof for the compositions of the present invention.

Carboxy methyl cellulose is the most preferred cellulosic gum/polymer for the compositions of the present invention.

Carboxymethyl cellulose (CMC) or cellulose gum is a cellulose derivative with carboxymethyl groups (-CH2-COOH) bound to some of the hydroxyl groups of the glucopyranose monomers that make up the cellulose backbone. It is often used as its sodium salt, sodium carboxymethyl cellulose.

Cellulosic type, controlled reaction parameters and conditions yield various technical grades of SCMC depending upon the application desired by the industry.

The structure is as follows.

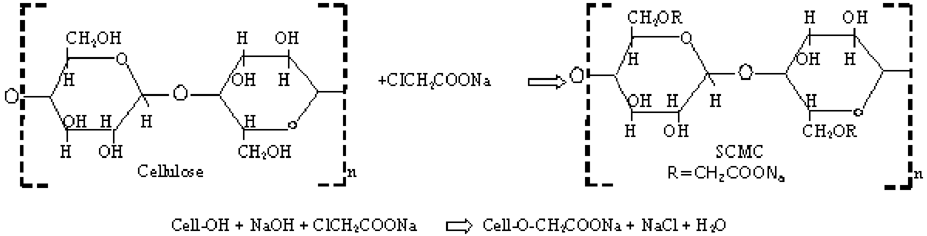

### Grade of cellulosic gum/polymer:

The selection of right grade of Cellulose Gum is very important else it will lead to Polymeric Gel settling/precipitation at bottom. Following parameters of cellulose Gum can impact water solubility, desire viscosity & gel settling: Degree of substitution, Degree of polymerization and impurity of organic salt eg. sodium glycolate.

### Degree of substitution:

The degree of substitution (D.S.), for e.g., in polymer carboxy methyl cellulose is the average number of carboxymethyl groups substituted per monomer unit, ranging from 0 to 3 with the remaining R = H. Aqueous solutions of NaCMC are generally complex. Some studies suggest that only a fraction is molecularly dispersed implying that NaCMC is a colloidally dispersed polymer. The degree of solubility is accepted to be a function of the degree of substitution, with less substituted (more hydrophobic) NaCMC showing a larger fraction of aggregates. Aggregated crystalline domains could be observed with degree of crystallinity correlating with lower D.S. and no crystallinity found for higher D.S. Additionally, low substitution grades could exhibit thixotropy which is thought to result from the formation of networks or soft aggregates. While with high D.S do not exhibit thixotropy as and, therefore, expect no significant blocks of unsubstituted cellulose, and thus no thixotropy or gelling at high concentrations.

The inventors of the present invention surprisingly found that when at most 50 wt% of the total weight of cellulosic polymer has degree of substitution less than 0.7 desired water solubility, viscosity was seen without any gel settling issue. It was further observed that, when the ratio of LDS and HDS is in the range of 1:1.5 to 1:4, there is a remarkable improvement in the stability and viscosity of the resultant liquid formulation.

At most 50 wt% of the total weight of cellulosic polymer has degree of substitution of less than 0.7 and more preferably less than 0.65 and most preferably less than 0.6.

It is further preferred that at most 50wt%, more preferably at most 45wt%, 43wt%, 40wt%, 38wt%, 37wt% by the weight of total polymer of the cellulosic polymer has degree of substitution less than 0.7.

It is further preferred that at least 1wt%, preferably at least 2wt%, 3wt%, 4wt%, 5wt%, 6wt%, 7wt%, 8wt%, 9wt%, 10wt%, 11wt%, 12wt%, 13wt%, 14wt%, 15wt%, 16wt%, 15wt%, 16wt%, 17wt%, 18wt%, 19wt%, 20wt%, of the cellulosic polymer by the weight of total polymer of the cellulosic polymer has degree of substitution less than 0.7.

It is preferred that 20 wt% to 50wt%, more preferably 22 to 45 wt% and most preferably 23 to 40 wt% of the the cellulosic polymer by the weight of total polymer of the cellulosic polymer has degree of substitution less than 0.7.

It is preferred that at least 50 wt% of the cellulosic polymer has degree of substitution higher than 0.75. It is preferred that 50 wt% to 80wt%, more preferably 55 to 78 wt% and most preferably 60 to 77 wt% of the the cellulosic polymer by the weight of total polymer of the cellulosic polymer has degree of substitution higher than 0.75.

It is further preferred that the cellulosic polymer having degree of substitution less than 0.7 and the cellulosic polymer having degree of substitution higher than 0.75, is in the range of 1:1.5 to 1:4, more preferably in the range of 1:2 to 1:3.5 and most preferably in the range of 1:2.5 to 1:3.

### Antibacterial activity and antimicrobial agent

The cleansing compositions include an antimicrobial agent which preferably is an antibacterial agent. The agent is primarily responsible for antibacterial action. Suitable antibacterial agents include 2-hydroxy-4,2',4'-trichlorodiphenylether (DP300); 2,6-dimethyl-4-hydroxychlorobenzene (PCMX); 3,4,4'-trichlorocarbanilide (TCC); 3-trifluoromethyl-4,4'-dichlorocarbaniide (TFC); 2,2'-dihydroxy-3,3',5,5',6,6'-hexachlorophenylmethane; 2,2'-dihydroxy-3,3', 5,5'-tetrachlorodiphenylmethane; 2,2'-dihydroxy-3,3',dibromo-5,5'-dichlorodiphenyl methane; 2-hydroxy-4,4'-dichlorodiphenylether; 2-hydroxy-3,5',4-tribromodiphenlylether; and 1-hydroxyl-4-methyl-6-(2,4,4-trimethylpentyl)-2(1H)-pyridinone (Octopirox), thymol and terpeniol. Particularly preferred antibacterial agents are thymol and terpeniol, optimally used in combination. In preferred compositions, the content of thymol ranges from 0.05 to 5 wt%, more preferably 0.1 to 1 wt% and most preferably 0.1 to 0.4 wt%. Above the preferred range, the compositions may have strong smell, which may not be preferred by some consumers. However, suitable strong masking agents liked perfumes can be used to mask the strong odour of thymol or terpeniol. As an alternative to thymol; thyme oil or thyme extract may also be added. Thyme oil or thyme extract is obtained from the thyme plant. Thyme plant refers to a plant belonging be genus Thymus and includes but is not limited to Thymus vulgaris, Thymus zygis, Thymus satureoides, Thymus mastichina, Thymus broussonetti, Thymus maroccanus, Thymus pallidus, Thymus algeriensis, Thymus serpyllum, Thymus pulegoide, and Thymus citriodorus .

A composite antimicrobial particulate material comprises an oligodynamic metal embedded in a water-insoluble inorganic carrier.

It is preferred that the inorganic carrier is at least one of titanium oxide, magnesium oxide, aluminium oxide, silicon oxide, calcium oxide, barium oxide, calcium hydroxyapatite, calcium carbonate, calcium magnesium carbonate, sheet silicate, zeolite, clay or bentonite. More preferably the inorganic carrier is composed of aggregates of nano plate like structures having width of 20 to 100 nm, preferably 40 to 60 nm. Further preferably the inorganic carrier is a porous material have a nano or micro-structured assembly. It is preferred that particle size of the inorganic carrier is 1 to 10 µm.

A particularly preferred inorganic carrier is calcium carbonate. Other preferred carriers are titanium oxide, magnesium hydroxide and zinc oxide.

Preferably the amount of oligodynamic metal comprised in the antimicrobial particulate material is from 0.1 to 10 wt% by weight of the particulate material. In such a case, the balance, more or less, is composed of the inorganic carrier. Suitable process conditions and stoichiometric amount of starting materials need to be employed to ensure that the antimicrobial particulate contains 0.1 to 10 wt% oligodynamic metal by weight of the particulate material. The process steps and conditions disclosed in WO16020168 A1 (Unilever) could be, and preferably are used to prepare the composite antimicrobial particulate material.

The process for preparing a composite antimicrobial particulate material comprises the following steps:
An aqueous dispersion of a water-insoluble inorganic carrier of particle size in the range of 1 to 10 µm is prepared. The dispersion contains 1 to 5 wt% (by weight of the dispersion) of the carrier.

Separately, an aqueous solution of a reducing agent is prepared. The concentration of the reducing agent in the solution is 10 to 30 wt%.

The dispersion and the solution are mixed. Thereafter, this mix is heated to 70 °C to 90 °C. At this stage, a water soluble salt of the oligodynamic metal is added to the heated mix.

Preferably, the reducing agent is sodium acetate, sodium oxalate, trisodium citrate or disodium ethylene diamine tetra acetate.

It is particularly preferred that the oligodynamic metal is copper or silver, furthermore particularly it is silver. Gold is also an alternative but is less preferred in view of its price. It is **preferred** that the compositions in accordance with this invention comprise an amount of the composite antimicrobial particulate equivalent to 0.001 to 2 wt% of the oligodynamic metal. For example, if it is desired that the composition should contain 0.5 wt% silver (= 0.5 g silver in 100 g of the composition), then an amount of the composite particulate material which contains 0.5 g silver is included in the composition. This amount could vary depending on the solvent content in the particulate material.

Silver is particularly preferred. In the ionic form it may exist as a salt or any compound in any applicable oxidation state. It is preferred that compositions in accordance with this invention comprise an amount of the composite antimicrobial particulate equivalent to 0.001 to 2 wt% of the oligodynamic metal. Where the metal is present in the form of a compound such as silver in the form of silver acetate; then an appropriate amount of the compound is included so that the active metal content is within the broad and preferred ranges as already indicated.

It is preferred that the metal, e.g. silver is present in the form of a compound, e.g. silver (I) compound but may also be in the form of particles, e.g. nanoparticles of silver.

Silver (I) compounds are one or more water-soluble silver(I) compounds having silver ion solubility at least 1 .0x10^{"4} mol/L (in water at 25 degrees C). Silver ion solubility, as referred to herein, is a value derived from a solubility product (Ksp) in water at 25
degrees centigrade, a well known parameter that is reported in numerous sources. More particularly, silver ion solubility [Ag+], a value given in mol/L may be calculated using the formula: [Ag+] = (Ksp^{▪} χ)^{(1/(χ+1 ))}, wherein Ksp is the solubility product of the compound of interest in water at 25 degrees centigrade, and x represents the number of moles of silver ion per mole of compound. It has been found that Silver(I) compounds having a silver ion solubility of at least 1 x 10^{"4} mol/L in are suitable for use herein. Silver ion solubility values for a variety of silver compounds are given in Table V.

**TABLE 1**

| Silver Ion Solubility |
|---|
| Silver Ksp /(mol/L in water at 25 X [Ag+] (mol/L in water at Compound °C) 25 °C). |
| Silver nitrate 1 51 .6 7.2 |
| Silver acetate 1 2.0 x 10-³ 4.5 x 10^{"2} |
| Silver sulfate 2 1 .4 x 10-⁵ 3.0 x 10^{"2} |
| Silver benzoate 1 2.5 x 10-⁵ 5.0 x 10-3 |
| Silver salicylate 1 1 .5 x 10-⁵ 3.9 x 10^{"3} |
| Silver carbonate 2 8.5 x 10-¹² 2.6 x 10^{"4} |
| Silver citrate 3 2.5 x 10-¹⁶ 1 .7 x 10^{"4} |
| Silver oxide 1 2.1 x 10-⁸ 1 .4 x 10^{"4} |
| Silver 3 8.9 x 10-¹⁷ 1 .3x 10^{"4} |
| phosphate |
| Silver chloride 1 1 .8 x 10-¹⁰ 1 .3 x 10^{"5} |
| Silver bromide 1 5.3 x 10-¹³ 7.3 x 10^{"7} |
| Silver iodide 1 8.3 x 10-¹⁷ 9.1 x 10^{"9} |
| Silver sulfide 2 8.0 x 10-⁵¹ 2.5 x 10^{"17} |

It is preferred that when silver is present in the form of a compound, the compound selected from silver oxide, silver nitrate, silver acetate, silver sulfate, silver benzoate, silver salicylate, silver carbonate, silver citrate or silver phosphate. In particularly preferred compositions the silver(I) compound is silver oxide.

### Thymol/Terpineol

In addition to the composite antimicrobial particulate material, the compositions in accordance with the invention comprise 0.0001 to 5 wt% of at least one of thymol or terpineol.

It is preferred that the compositions in accordance with this invention comprise thymol and terpineol, i.e. the two in combination. In such cases, it is preferred that the w/w ratio of the amount of thymol to that of terpineol is 1 :0.1 to 1 :10. More preferably the ratio is 1 :0.5 to 1 :2.

Where thymol is present alone or in combination with terpineol, it is preferred that amount of thymol is 0.01 to 2 wt%, more preferably 0.01 to 1 wt% and further more preferably 0.01 to 0.5 wt% and yet further most preferably 0.01 to 0.05 wt%. Thymol is efficacious at lower dosages therefore higher dosages may be used only if necessary.

A drawback of thymol is its odour which may appear unpleasant to some individuals but such higher dosages may be used in compositions where sensorial aspects are not important or in the case of the compositions which permit the inclusion of suitable odour masking agents. It is preferred that the thymol is used in purified form.

As an alternative to thymol, use may be made of thyme oil or thyme extract comprising thymol, while ensuring that sufficient/desired amount of thymol is present in it. Thyme oil or thyme extract is obtained from the thyme plant. Thyme plant refers to a plant belonging be genus Thymus and includes but is not limited to the following species: Thymus vulgaris, Thymus zygis, Thymus satureoides, Thymus mastichina, Thymus broussonetti, Thymus maroccanus, Thymus pallidus, Thymus algeriensis, Thymus serpyllum, Thymus pulegoide, and Thymus citriodorus. The isomer of thymol (carvacrol) may also be used. Alternatively, but less preferably, any derivative of thymol which has similar properties of thymol may also preferably be used.

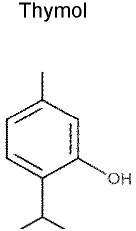

In addition to or instead of thymol, the compositions in accordance with this invention comprise terpineol. Where terpineol is present alone or in combination with thymol, it is preferred that amount of terpineol is 0.01 to 2 wt%, more preferably 0.01 to 1 wt% and further more preferably 0.01 to 0.5 wt% and yet further most preferably 0.01 to 0.05 wt%. Terpineol is efficacious at lower dosages therefore higher dosages may be used only if necessary.

A drawback of terpineol, like thymol, is its odour which may appear unpleasant to some individuals but such higher dosages may be used in compositions where sensorial aspects are not important or in the case of the compositions which permit the inclusion of suitable odour masking agents. It is preferred that the terpineol is used in purified form.

The structure of a terpineol is given below:

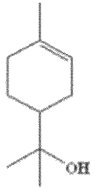

Alternatively, but less preferably, pine oil comprising desired amount of terpineol may be used instead of purified terpineol.

Oligodynamic metals, especially silver, are able to act largely due to its ability to affect the permeability of bacterial membranes and generate reactive oxygen species. Silver ions are considered as the active species. Therefore, if sufficient number of such ions are generated quickly and efficiently, it is possible to bring about rapid reduction in the viable bacterial count of surfaces which are contaminated therewith.

The oligodynamic metal, e.g. silver embedded in the inorganic carrier together with thymol (or terpineol or both) provides faster-acting antibacterial effect whilst permitting significant reduction in oligodynamic metal content that would otherwise have been necessary. The compositions in accordance with this invention are effective against at least one of Gram-positive and Gram-negative bacteria. More preferred compositions provide broad-spectrum antibacterial action which means action against Gram-positive and Gram-negative bacteria.

Without wishing to be bound by theory, it is believed that the inorganic carrier ensures the release of efficacious amount of ions of the oligodynamic metal. Such ions, together with at least one of thymol and terpineol, and preferably the two together; interact synergistically to provide 1 to 5 log reduction in bacterial count of an animate or inanimate surface at a contact time of 10 to 30 seconds.

### Optional Active Agents

Advantageously, active agents other than emollients defined above may be added to the cleansing composition in a safe and effective amount during formulation to treat the skin during the use of the product provided that they do not exceed solubility limits. Suitable active ingredients include those that are soluble in the aqueous phase. Suitable active agents may be advantageously selected from vitamins, anti-acne actives; anti-wrinkle, anti-skin atrophy and skin repair actives; skin barrier repair actives; non-steroidal cosmetic soothing actives; skin lightening actives; sunscreen actives; sebum stimulators; sebum inhibitors; anti-oxidants; protease inhibitors; skin tightening agents; desquamating enzyme enhancers; anti-glycation agents; topical anesthetics.

### Liquid Formulation

A liquid formulation is obtained by reconstituting the dry composition according to the first aspect with a suitable liquid carrier, the liquid composition comprising: 0.5 to 9 wt% of surfactant; 0.01 to 0.6wt% of chelating agent; 0.01 to 2 wt% preservative; cellulosic polymer, wherein at most 50 wt% of the total weight of cellulosic polymer has degree of substitution less than 0.7; and; and 85 to 97 wt% liquid carrier. In the liquid formulation the surfactant is present in the range of 0.5 to 9 wt%, more preferably in the range of 1 to 9 wt% and most preferably 2 to 9 wt%.

It is preferred that the liquid formulation further comprises a co-surfactant, present in the range of 0 to 2 wt%, more preferably in the range of 0.1 to 1.5 wt% and most preferably 0.3 to 1 wt%. In the liquid formulation the chelating agent is present in the range of 0.01 to 0.6 wt%, more preferably in the range of 0.05 to 0.5 wt% and most preferably 0.1 to 0.5 wt%.

In the liquid formulation the preservative is present in the range of 0.01 to 2 wt%, more preferably in the range of 0.1 to 2 wt% and most preferably 0.5 to 1 wt%.

In the liquid formulation the cellulosic polymer is present in the range of 0.1 to 3 wt%, more preferably in the range of 0.3 to 2 wt% and most preferably 0.4 to 1 wt%.

At most 50 wt% of the total weight of cellulosic polymer has degree of substitution of less than 0.7 and more preferably less than 0.65 and most preferably less than 0.6.

At most 50wt%, preferably at most 45wt%, 43wt%, 40wt%, 38wt%, 37wt% by the weight of total polymer of the cellulosic polymer has degree of substitution less than 0.7.

It is further preferred that at least 1wt%, preferably at least 2wt%, 3wt%, 4wt%, 5wt%, 6wt%, 7wt%, 8wt%, 9wt%, 10wt%, 11wt%, 12wt%, 13wt%, 14wt%, 15wt%, 16wt%, 15wt%, 16wt%, 17wt%, 18wt%, 19wt%, 20wt%, of the cellulosic polymer by the weight of total polymer of the cellulosic polymer has degree of substitution less than 0.7.

It is preferred that 20 wt% to 50wt%, more preferably 22 to 45 wt% and most preferably 23 to 40 wt% of the the cellulosic polymer by the weight of total polymer of the cellulosic polymer has degree of substitution less than 0.7.

It is preferred that at least 50 wt% of the cellulosic polymer has degree of substitution higher than 0.75. It is preferred that 50 wt% to 80wt%, more preferably 55 to 78 wt% and most preferably 60 to 77 wt% of the the cellulosic polymer by the weight of total polymer of the cellulosic polymer has degree of substitution higher than 0.75.

It is further preferred that the cellulosic polymer having degree of substitution less than 0.7 and the cellulosic polymer having degree of substitution higher than 0.75, is in the range of 1:1.5 to 1:4, more preferably in the range of 1:2 to 1:3.5 and most preferably in the range of 1:2.5 to 1:3.

The liquid carrier could be any suitable liquid carrier which allows dissolution of the components of the dry composition to form a stable flowable composition. It is most preferred that water is used as the liquid carrier.

It is preferred that the pH of the resultant liquid formulation made from reconstitution of the dry compositions of the present invention is in the range of 5.5 to 7 pH and more preferably in the range of 6 to 7 pH.

The viscosity of the liquid formulations of the present intention is in the range of 1000 to 4000 cps, more preferably 1500 to 3000 cps and most preferably between 1700 to 2500 cps.

### Use

The dry composition according to the first aspect is used to obtain a liquid formulation preferably the liquid formulation is obtained by reconstituting the dry composition with a suitable liquid carrier.

The liquid formulation made with the dry compositions of the present invention is used as a cleansing composition. More preferably the cleansing composition is a personal wash cleansing composition.

### Method

The dry composition of the present invention is prepared using the process comprising taking 30 to 90 wt% of surfactant; 1 to 6 wt% of chelating agent; 0.5 to 20 wt% preservative; and cellulosic polymer wherein at most 50 wt% of the total weight of cellulosic polymer has degree of substitution less than 0.7 and blending to form a mixture. It is preferred that to this mixture a benefit agent is added. It is preferred that the benefit agent is selected from the group of but not limited to a skin emollient, a skin care, a pharmaceutical benefit agent, an antibacterial agent, combinations and mixtures thereof.

The present invention also provides a method of making a liquid formulation from the dry composition according to the first aspect, the method comprising steps of, taking the dry composition and a container and diluting the dry composition with a liquid carrier with a dilution factor in the range of 1:10 to 1:50 to obtain the liquid formulation. It is more preferred that the dilution factor is in the range of 1:15 to 1:40 and most preferably 1:18 to 1:30.

It is preferred that the liquid carrier is water and more preferably clean potable water.

It is preferred that when the dry composition is packaged in the form of a sachet, such sachet is opened and poured into a container for reconstituting the dry composition to form a stable liquid cleansing formulation. It is preferred that the container is a bottle and most preferred a bottle with a dispenser such a s pump bottle. The bottle may first be filled with the liquid carrier such as water and then the dry composition added or vice versa. It is preferred that after the addition of the dry composition and the liquid carrier in a suitable container, the container is closed and shaken for about 15 to 60 seconds. It is preferred that the newly reconstituted liquid formulation is allowed to rest for at least 30 minutes and preferably for at least 1 to 2 hrs.

The invention will now be illustrated by means of the following non-limiting examples.

### Examples

### Example 1

### Preparation of formulations:

Sodium Lauryl Sulfate powder, Disodium Lauryl Sulfosuccinate powder, Tetrasodium EDTA, Sodium Benzoate were mixed in a ribbon blender for about 15-20 minutes. To this mixture silver oxide and thymol and terpineol as antibacterial agents, perfume and colorant was added.

The Liquid formulation was formed by taking about 9 gm of dry composition and mixing it with 200ml water in a pump bottle, shaking the bottle for about 30 to 60 seconds and allowing it to rest for about 1 to 2 hrs before use.

**Table 1**

| **Ingredients** | **Wt%** |
|---|---|
| Sodium Lauryl Sulfate | 50 |
| Disodium Lauryl Sulfosuccinate | 11 |
| Di sodium EDTA (Powder) | 4 |
| Cellulose Gum (HDS) - degree of substitution- 0.8 (68.75wt%) | 11 |
| Cellulose Gum (LDS) - degree of substitution- 0.6 (31.25wt%) | 5 |
| Potassium Sorbate | 15 |
| Silver-DTPA solution | 0.2 |
| Minors made up to 100wt% | 100 |

### Example 2

Different dry compositions P1 to P7 were prepared with differing wt% of cellulose gum and differing combinations of polymer substitution in the dry composition according to Example 1 and differing ratio of LDS and HDS were tested for stability and desired viscosity as between 1000- 4500cps being medium viscous.

The degree of substitution can be measured by the standard method of (ASTM D1439-03, 2008).

**Table 2:**

| Dry Composition code name* | Degree of substitution | | Observations at To | at T₀₊₃₀ₘᵢₙ. | | at T₀₊₁₂₀ₘᵢₙ. | |
|---|---|---|---|---|---|---|---|
| | | % of LDS/HDS (Ratio) | | Visco sity | Observations | Observ ations | Viscosity |
| P1- 17wt% cellulose gum | 0.55 to 0.64 | 100% LDS (1) | Undissolved polymeric lumps seen & no viscosity build up | 820 | Gel settled at bottom. | 760 | Gel settled at bottom. |
| P2- 17wt% cellulose gum | <0.5 | 100%LDS (1) | Undissolved polymeric lumps seen & no viscosity build up | 1680 | Gel settled at bottom. | 1680 | Gel settled at bottom. |
| P3- 16wt% cellulose gum | LDS- 0.55 to 0.64 | 25LDS%; | Undissolved polymeric lumps seen & no viscosity build up | 1580 | Clear Solution, no Gel settled at bottom. | 1700 | Clear Solution, no Gel settled at bottom. |
| | HDS- 0.85 | 75HDS% (1:3) | | | | | |
| P4- 19wt% cellulose gum | <0.6 | 100%LDS (1) | Undissolved polymeric lumps seen & no viscosity build up | 2780 | Gel settled at bottom. | 2780 | Gel settled at bottom. |
| P5- 14wt% cellulose gum | LDS- 0.55 to 0.64 | 35LDS%; | Undissolved polymeric lumps seen & no viscosity build up | 1400 | Clear Solution, no Gel settled at bottom. | 1420 | Clear Solution, no Gel settled at bottom. |
| | HDS- 0.85 | 65HDS% (1:1.8) | | | | | |
| P-6 16% Cellulose Gum | LDS- 0.55 to 0.64 | 30LDS%; | Undissolved polymeric lumps seen & no viscosity build up | 2000 | Clear Cleanser Solution, no Gel settled at bottom | 2320 | Clear Cleanser Solution, no Gel settled at bottom |
| | HDS- 0.85 | 70HDS% (1:2.2) | | | | | |
| P-7 12% Cellulose Gum | LDS- 0.55 to 0.64 | 42LDS% : | Some undissolved polymeric lump | 940 | No Gel settling at bottom, some undissolved polymeric Lumps were observed | 1000 | No Gel settling at bottom, some undissolved polymeric Lumps were observed |
| | | 58HDS% (1:1.4) | | | | | |
| | HDS- 0.85 | | | | | | |

Data in table 1 show that when different cellulosic gums when only changed in ratio of LDS and HDS show better stability and desired viscosity. It is seen that when the same polymer is 100% LDS as in P1 and P2, it shows gel settling and undesirable viscosity. However, when the ratio of LDS and HDS is in the range of 1:1.5 to 1:4, such as in P3, P5-P7, there is a remarkable improvement in the stability and viscosity of the resultant liquid formulation.

Therefore, it can be concluded that the ratio of LDS and HDS of the cellulosic polymer plays an important role in the desirable characteristics and stability of the reconstituted resultant liquid formulation.

## Claims

1. A dry composition suitable for forming stable liquid cleansing compositions comprising:
i) 30 to 90 wt% of surfactant;
ii) 1 to 6 wt% of chelating agent;
iii) 0.5 to 20 wt% preservative; and
iv) cellulosic polymer, wherein at most 50 wt% of the total weight of cellulosic polymer has degree of substitution less than 0.7.

2. A composition according to claim 1, wherein the surfactant is in the range of 40 to 80 wt% of the dry composition.

3. A composition according to claim 1 or 2, wherein the surfactant is an anionic surfactant

4. A composition according to anyone of the preceding claims 1 to 3, wherein the surfactant comprises co-surfactant up to 20 wt% of the dry composition.

5. A composition according to anyone of the preceding claims 1 to 4, wherein the chelating agent is in the range of 1.5 to 5.5 wt%.

6. A composition according to anyone of the preceding claims 1 to 5, wherein the chelating agent is selected from the group of Ethylene Diamine Tetra Acetic acid (EDTA), or Diethylene Triamine Penta Acetic acid (DTPA), combinations and mixtures thereof.

7. A composition according to anyone of the preceding claims 1 to 6, wherein the preservative is in the range of 1.5 to 18 wt% of the dry composition.

8. A composition according to anyone of the preceding claims 1 to 7, wherein the preservative is selected from sodium benzoate, benzoic acid, potassium sorbate, methylparaben, ethylparaben, propylparaben, butylparaben and heptyl paraben, combinations or mixtures thereof.

9. A composition according to anyone of the preceding claims 1 to 8, wherein at least 60 wt% of the cellulosic polymer has degree of substitution higher than 0.75.

10. A composition according to claim 9, wherein the cellulosic polymer having degree of substitution less than 0.7 and the cellulosic polymer having degree of substitution higher than 0.75, is in the range of 1:1.5 to 1:4.

11. A composition according to anyone of the preceding claims 1 to 10, wherein the cellulosic polymer is in the range of 5 to 30 wt% of the dry composition.

12. A composition according to anyone of the preceding claims 1 to 11, wherein the cellulosic polymer is a carboxy methyl derived cellulose.

13. A method of making a liquid formulation from the dry composition according to anyone of the preceding claims 1 to 12 the method comprising steps of, taking the dry composition and a container and diluting the dry composition with a liquid carrier with a dilution factor in the range of 1:10 to 1:50 to obtain the liquid formulation.

## Patentansprüche

1. Trockene Zusammensetzung, die zur Bildung stabiler flüssiger Reinigungszusammensetzungen geeignet ist, umfassend:
i) 30 bis 90 Gew.-% Tensid;
ii) 1 bis 6 Gew.-% Chelatbildner;
iii) 0,5 bis 20 Gew.-% Konservierungsmittel; und
iv) Cellulosepolymer, wobei höchstens 50 Gew.-% des Gesamtgewichts des Cellulosepolymers einen Substitutionsgrad von weniger als 0,7 aufweisen.

2. Zusammensetzung nach Anspruch 1, wobei das Tensid im Bereich von 40 bis 80 Gew.-% der trockenen Zusammensetzung vorliegt.

3. Zusammensetzung nach Anspruch 1 oder 2, wobei das Tensid ein anionisches Tensid ist.

4. Zusammensetzung nach irgendeinem der vorhergehenden Ansprüche 1 bis 3, wobei das Tensid bis zu 20 Gew.-% der trockenen Zusammensetzung Co-Tensid umfasst.

5. Zusammensetzung nach irgendeinem der vorhergehenden Ansprüche 1 bis 4, wobei der Chelatbildner in dem Bereich von 1,5 bis 5,5 Gew.-% vorliegt.

6. Zusammensetzung nach irgendeinem der vorhergehenden Ansprüche 1 bis 5, wobei der Chelatbildner aus der Gruppe von Ethylendiamintetraessigsäure (EDTA) oder Diethylentriaminpentaessigsäure (DTPA), Kombinationen und Mischungen davon ausgewählt ist.

7. Zusammensetzung nach irgendeinem der vorhergehenden Ansprüche 1 bis 6, wobei das Konservierungsmittel in dem Bereich von 1,5 bis 18 Gew.-% der trockenen Zusammensetzung vorliegt.

8. Zusammensetzung nach irgendeinem der vorhergehenden Ansprüche 1 bis 7, wobei das Konservierungsmittel aus Natriumbenzoat, Benzoesäure, Kaliumsorbat, Methylparaben, Ethylparaben, Propylparaben, Butylparaben und Heptylparaben, Kombinationen oder Mischungen davon ausgewählt ist.

9. Zusammensetzung nach irgendeinem der vorhergehenden Ansprüche 1 bis 8, wobei mindestens 60 Gew.-% des Cellulosepolymers einen Substitutionsgrad von über 0,75 aufweisen.

10. Zusammensetzung nach Anspruch 9, wobei das Cellulosepolymer mit einem Substitutionsgrad von weniger als 0,7 und das Cellulosepolymer mit einem Substitutionsgrad von über 0,75 in dem Bereich von 1:1,5 bis 1:4 vorliegen.

11. Zusammensetzung nach irgendeinem der vorhergehenden Ansprüche 1 bis 10, wobei das Cellulosepolymer in dem Bereich von 5 bis 30 Gew.-% der trockenen Zusammensetzung vorliegt.

12. Zusammensetzung nach irgendeinem der vorhergehenden Ansprüche 1 bis 11, wobei das Cellulosepolymer eine von Carboxymethylcellulose abgeleitete Cellulose ist.

13. Verfahren zur Herstellung einer flüssigen Formulierung aus der trockenen Zusammensetzung nach irgendeinem der vorhergehenden Ansprüche 1 bis 12, wobei das Verfahren die Schritte umfasst, bei denen die trockene Zusammensetzung und ein Behälter genommen und die trockene Zusammensetzung mit einem flüssigen Träger mit einem Verdünnungsfaktor in dem Bereich von 1:10 bis 1:50 verdünnt wird, um die flüssige Formulierung zu erhalten.

## Revendications

1. Composition sèche convenant à la formation de compositions nettoyantes liquides stables comprenant:
i) 30 à 90 % en poids de tensioactif;
ii) 1 à 6 % en poids d'agent chélateur;
iii) 0,5 à 20 % en poids de conservateur; et
iv) un polymère cellulosique, où au plus 50 % en poids du poids total du polymère cellulosique a un degré de substitution inférieur à 0,7.

2. Composition selon la revendication 1, où le tensioactif est dans la plage de 40 à 80 % en poids de la composition sèche.

3. Composition selon la revendication 1 ou 2, où le tensioactif est un tensioactif anionique.

4. Composition selon l'une quelconque des revendications 1 à 3 précédentes, où le tensioactif comprend un co-tensioactif jusqu'à 20 % en poids de la composition sèche.

5. Composition selon l'une quelconque des revendications 1 à 4 précédentes, où l'agent chélateur est dans la plage de 1,5 à 5,5 % en poids.

6. Composition selon l'une quelconque des revendications 1 à 5 précédentes, où l'agent chélateur est choisi dans le groupe de l'acide éthylènediaminetétraacétique (EDTA) ou de l'acide diéthylène-triaminepentaacétique (DTPA), leurs combinaisons et leurs mélanges.

7. Composition selon l'une quelconque des revendications 1 à 6 précédentes, où le conservateur est dans la plage de 1,5 à 18 % en poids de la composition sèche.

8. Composition selon l'une quelconque des revendications 1 à 7 précédentes, où le conservateur est choisi parmi le benzoate de sodium, l'acide benzoïque, le sorbate de potassium, le méthylparabène, l'éthylparabène, le propylparabène, le butylparabène et l'heptylparabène, leurs combinaisons ou leurs mélanges.

9. Composition selon l'une quelconque des revendications 1 à 8 précédentes, où au moins 60 % en poids du polymère cellulosique a un degré de substitution supérieur à 0,75.

10. Composition selon la revendication 9, où le polymère cellulosique ayant un degré de substitution inférieur à 0,7 et le polymère cellulosique ayant un degré de substitution supérieur à 0,75 est dans la plage de 1:1,5 à 1:4.

11. Composition selon l'une quelconque des revendications 1 à 10 précédentes, où le polymère cellulosique est dans la plage de 5 à 30 % en poids de la composition sèche.

12. Composition selon l'une quelconque des revendications 1 à 11 précédentes, où le polymère cellulosique est une cellulose dérivée par carboxyméthyle.

13. Procédé de production d'une formulation liquide à partir de la composition sèche selon l'une quelconque des revendications 1 à 12 précédentes, le procédé comprenant les étapes de prendre la composition sèche et un récipient et diluer la composition sèche avec un véhicule liquide avec un facteur de dilution dans la plage de 1:10 à 1:50 pour obtenir la formulation liquide.
